# EUROPEAN PATENT SPECIFICATION

(11) **EP 4 301 441 B1**
(45) Date of publication and mention of the grant of the patent: **11.11.2026**
(21) Application number: 23756029.7
(22) Date of filing: 21.02.2023
(51) Int. Cl.: A61M 15/00, A61M 11/00, A61M 11/06, A61M 16/08, B05B 1/28, A61M 16/06, A61M 11/08, B05B 7/24

(54) **SYSTEM FOR PROVIDING A NON-ELECTRIC NEBULIZER**
SYSTEM ZUR BEREITSTELLUNG EINES NICHTELEKTRISCHEN VERNEBLERS
SYSTÈME POUR FOURNIR UN NÉBULISEUR NON ÉLECTRIQUE

(30) Priority: 21.02.2022 IN 202211009115
(43) Date of publication of application: 10.01.2024
(73) Proprietor: UCS Wellness Private Limited, Ghaziabad 201014 (IN)
(72) Inventor: SHEEL, Umang Dev, Uttar Pradesh Ghaziabad 201014 (IN); SHEEL, Ananth, Uttar Pradesh Ghaziabad 201014 (IN); SAXENA, Alankar, Uttar Pradesh Ghaziabad 201014 (IN)
(74) Representative: Vossius & Partner Patentanwälte Rechtsanwälte mbB
(86) International application number: PCT/IN2023/050167
(87) International publication number: WO 2023/157029

(56) References cited:
- EP-A1- 3 323 455
- WO-A2-2006/006963
- US-A- 3 658 059
- US-A- 5 586 551
- US-A1- 2001 035 181
- US-A1- 2018 008 789
- US-B1- 9 511 202

## Description

### TECHNICAL FIELD

The present disclosure generally relates to the field of the fluid dynamics devices, and more particularly to a method and system for providing a non-electric nebulizer.

### BACKGROUND

The following description of the related art is intended to provide background information pertaining to the field of the disclosure. This section may include certain aspects of the art that may be related to various features of the present disclosure. However, it should be appreciated that this section is used only to enhance the understanding of the reader with respect to the present disclosure, and not as admission of the prior art.

It is known that a nebulizer for the inhalation of fluid requires electricity to run the nebulizer and thus with respect to its mode of operation, it must be plugged into an electrical socket for use. However, sometimes the user of the nebulizer may not have any access to the electrical sockets and therefore, if the user needs to use the nebulizer outdoors or away from the electrical socket, then the user will not be able to do so, as nebulizers can be functional only when connected to electric power. Further, the dependency of the nebulizer on the power limits the use of the nebulizer. Therefore, the user can't use the nebulizer if the user is not in the vicinity of an electric power supply system. Moreover, the conventionally available nebulizers are inconvenient for the users to inhale fluid from the nebulizer mask if the body posture is not appropriate or the user is lying in different postures.

Hence, in view of these and other existing limitations, there arises an imperative need to provide an efficient solution to overcome the above-mentioned limitations and to provide a method and system to allow the use of the nebulizer by the user in any required posture without the need of electric power supply.

US 3658059 A relates to inhalers for producing suspensions of liquid or pulverulent materials. EP 3323455 A1 relates to an inhalation device for the purpose of inhaling a mist of droplets by a user.

WO 2006/006963 relates to systems for aerosol delivery of agents to a patient.

US 2018/008789 A1 relates to an apparatus for providing a nebula or aerosol to a patient. US 9511202 B1 relates to a breathing assistance device with nebulizer.

### SUMMARY

This section is provided to introduce certain objects and aspects of the present disclosure in a simplified form that are further described below in the detailed description. This summary is not intended to identify the key features or the scope of the claimed subject matter.

The invention is specified by the independent claims. Preferred embodiments are defined in the dependent claims. In the following description, although numerous features may be designated as optional, it is nevertheless acknowledged that all features comprised in the independent claims are not to be read as optional.

The present disclosure provides a non-electric nebulizer system. The system includes at least one container, at least one valve, at least one mask holder, at least one mask outer case, at least one Bernoulli's Theory Enabler, at least one fluid tank, at least one disbursement trigger, and at least one pipe tube point. The valve is air tightly positioned in the container. The mask holder is positioned on the top of the at least one valve. The mask outer case is fixed on top of the mask holder and the Bernoulli's Theory Enabler is positioned inside the fluid tank. The fluid tank is positioned on the top of the at least one mask holder. The disbursement trigger is fixed on top of the at least one mask outer case. In an embodiment, the non-electric nebulizer system is activated based on the movement of the at least one mask outer case in a clockwise direction and deactivated based on the movement of the at least one mask outer case in the anti-clockwise direction for a predefined number of rotations.

In an embodiment of the present disclosure, the non-electric nebulizer system is activated by pressing the disbursement trigger of the mask and deactivated by releasing the disbursement trigger of the mask.

The present disclosure further provides a method for inhaling fluid using a non-electric nebulizer. The method includes receiving a first input from a user for activation of the non-electric nebulizer. Next, the method includes the step of activating the non-electric nebulizer by initiating the movement of the fluid from at least one fluid tank to at least one mask outer case using Bernoulli's Theory Enabler. Next, the method includes the step of inhaling the fluid by the user using at least one mask outer case of the non-electric nebulizer. Next, the method includes the step of receiving a second input from the user for the deactivation of the non-electric nebulizer. Thereafter, the method includes the step of deactivating the non-electric nebulizer by stopping the movement of the fluid from at least one fluid tank based on the second input.

### BRIEF DESCRIPTION OF THE DRAWINGS

The accompanying drawings, which are incorporated herein, and constitute a part of this disclosure, illustrate exemplary embodiments of the disclosed methods and systems in which like reference numerals refer to the same parts throughout the different drawings. Components in the drawings are not necessarily to scale, emphasis instead being placed upon clearly illustrating the principles of the present disclosure. Some drawings may indicate the components using block diagrams and may not represent the internal circuitry of each component. It will be appreciated by those skilled in the art that disclosure of such drawings includes disclosure of electrical components, electronic components or circuitry commonly used to implement such components.
FIG.1 illustrates a section view diagram of the system [100] for providing a non-electric nebulizer system, in accordance with exemplary embodiment of the present disclosure.
FIG.2 illustrates a top view diagram of the system [100] for providing the non-electric nebulizer system, in accordance with exemplary embodiment of the present disclosure.
FIG.3 illustrates a side view diagram of the system [100] for providing the non-electric nebulizer system, in accordance with exemplary embodiment of the present disclosure.
FIG.4 illustrates a schematic diagram of the system [100] for providing the non-electric nebulizer system integrated with a tube pipe for inhalation of fluid by the user in any posture, in accordance with exemplary embodiment of the present disclosure.
FIG.5 illustrates a method flow diagram [200] for inhaling fluid using a non-electric nebulizer, in accordance with the exemplary embodiment of the present disclosure.

The foregoing shall be more apparent from the following more detailed description of the disclosure.

### DETAILED DESCRIPTION

In the following description, for the purposes of explanation, various specific details are set forth in order to provide a thorough understanding of embodiments of the present disclosure. It will be apparent, however, that embodiments of the present disclosure may be practiced without these specific details.

An individual feature may not address any of the problems discussed above or might address only some of the problems discussed above. Some of the problems discussed above might not be fully addressed by any of the features described herein.

As discussed in the background section, conventionally available nebulizers do not allow users to use the nebulizer for the inhalation of fluid like medicinal drugs, and oxygen without connecting the nebulizer to an electric power supply source. The conventionally available nebulizers require connection with electric power for initiating the flow of gas from the fluid tank of the nebulizer to the mask, for the inhalation of fluid when the user wears the mask. Therefore, the user is not able to use the conventionally available nebulizers when he is outdoors or away from the power supply, thus leading to the inefficient use of the nebulizer system. Further, conventionally available systems are not appropriate for the user who want to use the nebulizer system in any desired posture.

To overcome the problems as mentioned in the background section, an object of the present disclosure is to provide a system for using a nebulizer without electric power. It is yet another object of the present disclosure to enable the user to use the nebulizer outdoors or any area that is away from the electric power supply. Yet another object of the present disclosure is to provide non-electric nebulizer for the continuous or non-continuous inhalation of fluid such as oxygen or liquid medicines. It is yet another object of the present disclosure to enable the user to use the nebulizer for the inhalation of fluids in any body posture as per the comfortability of the user.

To achieve the aforementioned objectives, the present disclosure provides a method and system for the continuous and non-continuous flow of gas or mixture of gases. For this purpose, a gas-pressure based mechanism using the Bernoulli's theorem is used to use the nebulizer without any external electric or mechanical power source. One aspect of the present disclosure relates to a system for providing a non-electric power nebulizer system. The system includes but is not limited to a bottle, a valve, a mask holder, a mask outer case, a Bernoulli's Theory Enabler, a fluid tank, and a disbursement trigger that are operatively connected to form a non-electric nebulizer. The non-electric nebulizer user the gas or mixture of gases stored in the container to enable the function of the nebulizer by eliminating the need of electric power. Further, the present disclosure provides the method and system to connect the non-electric power nebulizer with an external mask via a tube point to enable the user to use the nebulizer mask for the inhalation of fluid in any favourable posture of the body.

The present disclosure provides a solution relating to the inhalation of the medicinal drug or fluid through a non-electric nebulizer system. More specifically, the present disclosure provides a system for providing a nebulizer that works without electric power and also allows the user to inhale fluids directly from the connected non-electric nebulizer outer mask or an external mask connected via a tube pipe. The present disclosure uses Bernoulli's Theory Enabler component in the fluid tank and the mask holder of the non-electric nebulizer system to make the nebulizer independent of the electric power supply. The present disclosure also includes a feature to use a pipe that connects the external mask to the non-electric nebulizer for allowing the user to inhale the fluid in any favourable body postures.

As used herein, "fluid", may be any substance of matter that may include but is not limited to liquids, or gases, or a mixture of liquids, or a mixture of gases, or a mixture of liquids and gases stored under pressure in the valve container. Further, in the specification, the terms "fluid" and "gas" and "mixture of gases or air" can be used interchangeably as well.

As used herein, "bottle", "container", "tank" referred to a component in any preferred shape of a particular volume that holds the fluid under compressed pressure.

As used herein, "valve", referred to a component with a pipe-like structure. The valve is positioned air tightly inside the "bottle" or "container" or "tank" containing gas under compressed pressure for the delivery of the gas to the mask of the non-electric nebulizer system.

As used herein, "mask holder", referred to a component of any required shape that is positioned at the upper side of the valve for holding the mask firmly with the help of built-in internal grooves for the delivery of the gas to the mask of the non-electric nebulizer system.

As used herein, "mask outer case", referred to a component of preferably conical shape to cover the nose and/or mouth that is positioned at the top region of the mask holder. The mask outer case may be connected to the disbursement trigger for movement of the mask outer case in a clockwise and anti-clockwise direction to control the delivery of the fluid to the mask of the non-electric nebulizer system or by continuously pressing the trigger for the same purpose. In addition, movement of the mask outer case in the clockwise and anti-clockwise direction helps in adjusting the pressure of the air or gas or mixture of gases through the valve.

As used herein, "Bernoulli's Theory Enabler", referred to a component that is positioned inside the fluid tank of the non-electric nebulizer system at the top region of the bottle or container for the delivery of the gas or air or mixture of gases to the mask of the non-electric nebulizer system in compliance with Bernoulli's Theorem of fluid dynamics. Further, with the release of the high pressure of air or gas or mixture of gases, the fluid from the fluid tank gets activated to disburse the fluid to the mask outer case.

As used herein, "fluid tank", referred to a component that is positioned at the top region of the mask holder with its lower base outlet positioned on top of the valve of the bottle or container for the storage of air or gas or mixture of gases and dispensing of the stored fluid for the delivery of the fluid to the mask of the non-electric nebulizer system in compliance with Bernoulli's Theorem of fluid dynamics.

As used herein, "disbursement trigger", referred to a component that is connected with the mask outer case for disbursement of high-pressure gas or air or mixture of gases. The delivery of the fluid from the fluid container to the mask of the non-electric nebulizer system is achieved by continuously pressing the disbursement trigger. The non-electric nebulizer system is activated by pressing the disbursement trigger of the mask and deactivated by releasing the disbursement trigger of the mask.

As used herein, "tube pipe", referred to a component that connects the non-electric nebulizer system to the external nebulizer mask for allowing the user to inhale fluids from the nebulizer mask in any body posture.

As used herein, "preferred number of rotations", referred to number of rotation required for minimal disbursement of the high pressure of the gas or air or mixture of gases to activate and deactivate the nebulizer.

As used herein, "external mask", referred to external nebulizer mask for allowing the user to inhale fluids from the nebulizer mask in any body posture.

As used herein, "dip tube" or "dip pipe" referred to a tube of which the top end is connected to the at least one valve. Another end of the dip tube is open in the container through which the fluid flows.

The present disclosure is further explained in detail below with reference to the diagrams.

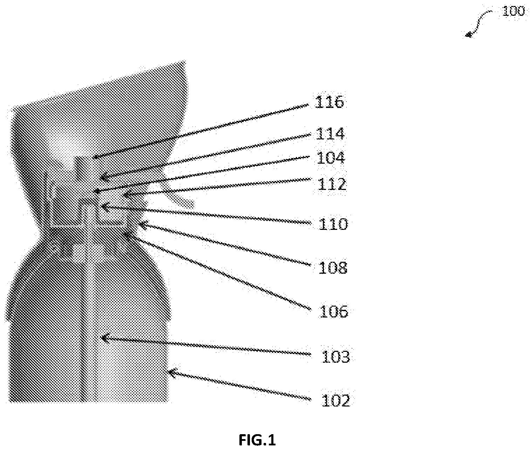

FIG.1 illustrates a sectional view diagram of the system [100] for providing a non-electric nebulizer system, in accordance with the exemplary embodiment of the present disclosure. As shown in Fig. 1, the system [100] includes at least one bottle or one container [102], at least one valve [104], at least one mask holder [106], at least one mask outer case [108], at least one Bernoulli's Theory Enabler [110], at least one fluid tank [112], at least one Disbursement Trigger [114], and at least one pipe tube point [116], wherein all the components are assumed to be connected to each other unless otherwise indicated below. The system [104] also includes at least one dip tube [103] connected to the at least one valve [104]. Also, in Fig. 1 only one bottle or one container [102], only one dip tube [103], only one valve [104], only one mask holder [106], only one mask outer case [108], only one Bernoulli's Theory Enabler [110], only one fluid tank [112], and only one Disbursement Trigger [114] and only one tube point [116] is shown, however, the system [100] may comprise multiple such units and modules or the system may comprise any such numbers of said units and modules, as may be required to implement the features of the present disclosure. Also, there may be one or more sub-units of said units and modules of the system [100] and the same is not shown in the Fig. 1 for the purpose of clarity.

The present disclosure provides a non-electric nebulizer system [100]. The system includes at least one container [102], at least one valve [104] with at least one dip tube [103] air tightly positioned in the at least one container [102], at least one mask holder [106] positioned on the top of the at least one valve [104], at least one mask outer case [108] fixed on top of the at least one mask holder [106], at least one Bernoulli's Theory Enabler [110] positioned inside the at least one fluid tank [112], the at least one fluid tank [112] positioned on the top of the at least one mask holder [106], at least one disbursement trigger [114] fixed on top of the at least one mask outer case [108], and at least one pipe tube point [116].

In an embodiment, the non-electric nebulizer system [100] is activated based on the movement of the at least one mask outer case [108] in a clockwise direction for a predefined number of rotations. In another embodiment, the non-electric nebulizer system is deactivated based on the movement of the mask outer case [108] in anti-clockwise direction for the predefined number of rotations. In a non-limiting embodiment, the at least one pipe tube point [116] enables the user to connect the non-electric nebulizer system with an external mask to use the non-electric nebulizer system in one or more favourable positions of the user.

In a non-limiting embodiment, the non-electric nebulizer system is further activated by pressing the disbursement trigger [114] of the mask and deactivated by releasing the disbursement trigger [114] of the mask. The system includes the bottle or container [102]. In the present disclosure, the bottle [102] is a container [102] that contains fluids such as liquid, gas or a mixture of gases or compressed air to perform the non-electric nebulizer therapy. For instance, the bottle or container [102] may contain gases or fluid or compressed air or mixture of gases to inhale them with medicine or taking steam and the like.

The system includes the valve [104]. The valve [104] with the dip pipe [103] is air tightly positioned in the bottle [102]. The valve [104] act as threshold against the air to adjust the flow of fluid using a flow controller.

In a non-limiting embodiment, the flow controller is positioned to encapsulate the dip pipe of the valve [104] of the non-electric nebulizer system to enable the controlling of the flow of the fluid through the valve [104]. In an example, the flow controller act as a jacket of the dip pipe of the valve to vary the flow of the fluid through the dip pipe. Further, the flow controller controls the inflow and outflow of fluid or gas or mixture of gases or air. The flow controller attached to the bottom end of the dip-tube of the valve that remains inside the bottle or the container to reduce the area and increase the pressure of the fluid passing through the dip pipe or dip tube of the non-electric nebuliser.

Further, the valve [104] is connected to the mask holder [106] at the top that positions the mask outer case [108] firmly for the user to inhale fluid by using the mask of the non-electric nebulizer system. The mask holder [106] holds the mask outer case [108] firmly with a plurality of built-in grooves that ensure the firm positioning and movement of the mask outer case [108]. The connection of the mask holder [106] with the mask outer case [108] enables the movement of the mask outer case [108] in a clockwise and anti-clockwise direction for enabling the functioning of the non-electric nebulizer.

The mask holder [106] is connected to the fluid tank [112] that is positioned on the top of the valve [104] and is used for storing and dispensing the stored fluid such as medicinal drug. The fluid tank [112] is fitted in with the Bernoulli's Theory Enabler [110] that is placed inside the fluid tank [112]. The Bernoulli's Theory Enabler [110] component gets activated upon the release of the high pressure of the gas or air or mixture of gases that makes the gas or air or mixture of gases flow continuously from the fluid tank [112] to the mask outer case [108] in adherence with Bernoulli's Theorem of fluid dynamics. In fluid dynamics, Bernoulli's principle states that an increase in the speed of a fluid occurs simultaneously with a decrease in static pressure or a decrease in the fluid's potential energy. The mask outer case [108] of the system is connected with the disbursement trigger [114] that enables the disbursement of the high pressure of the gas or air or mixture of gases to make the user inhale the gas or air or mixture of gases while wearing the external mask of the non-electric nebulizer system. The lower part of the disbursement trigger [114] mixes the high-pressure gases or mixture of gases from the bottle [102] with the stored fluid of the fluid tank [112] through Bernoulli's Theory Enabler [110] as an outlet through the mask outer case [108] fixed on the top of the mask holder. Further, the disbursement trigger [114] works by mixing the high-pressure air/gas or fluid of the bottle [102] with the pressure of the stored fluid in the fluid tank [112] to disburse the continuous or non-continuous flow of the gases or mixtures in a controlled manner. Finally, the flow of the gases or mixture moves in an upward direction from the fluid tank [112] to the mask outer case [108] enable the user to inhale the fluid through the mask outer case.

The disbursement trigger [114] is connected with at least one tube point [116] of the mask. At least one tube point [116] enables the user to connect the non-electric nebulizer system with an external mask to take the benefit of the non-electric nebulizer system in any suitable or favourable position.

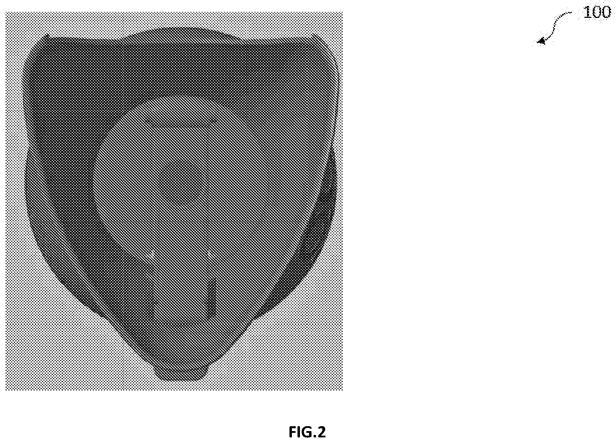

Fig. 2 illustrates a top view of the non-electric nebulizer system, in accordance with exemplary embodiment of the present disclosure. Fig. 2 illustrates the top view of the system [100] with all the components as mentioned in the above Fig. 1.

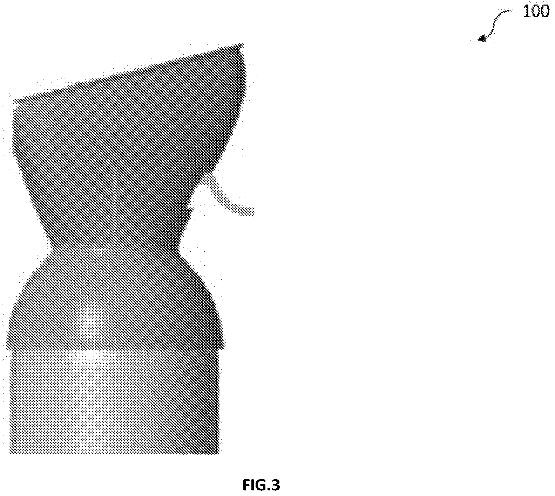

Fig. 3 illustrates the side view of the non-electric nebulizer system with all the components as mentioned in the above Fig. 1.

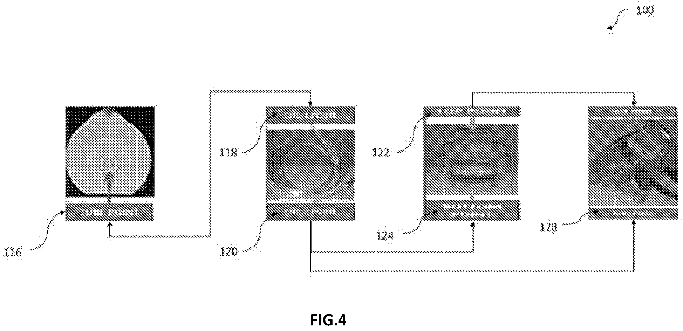

FIG.4 illustrates a schematic diagram of the system [100] for providing the non-electric nebulizer system integrated with a tube pipe for the inhalation of fluid by the user in any favourable posture, in accordance with the exemplary embodiment of the present disclosure. In an embodiment of the present disclosure, the at least one tube point [116] of the non-electric nebulizer mask is connected to the first end point [118] of the at least one pipe. Further, the second end point [120] of the at least one pipe is operatively connected to the bottom point [124] of the at least one external nebulizer fluid tank. Next, the top point [122] of the at least one external nebulizer fluid tank [112] is connected to the tube point [116] of the external mask to allow the user to inhale fluid from the external nebulizer mask in any posture of the body. In another embodiment of the present disclosure, the at least one tube point [116] of the non-electric nebulizer mask is connected to the first end point [118] of the at least one pipe and the second end point [120] of the at least one pipe is connected with bottom point [124] of external nebulizer tank or directly connected with the tube point [116] of the external nebulizer mask to allow the user to inhale fluid from the external mask in any suitable or favourable position. In an example, the user keeps the non-electric nebulizer system on the table and directly inhale the fluid or medicine from the mask of the non-electric nebulizer by sitting on the chair without using any external pipe and external nebulizer mask. In another example, the user can directly use the non-electric nebulizer while traveling without the hassle of keeping multiple attachments for inhaling the fluid. In yet another example, the user is asleep on a bed or reclined in any favourable position of the body. During this, the user is using the external nebulizer mask to inhale medicines in the fluid form contained in the fluid tank [112] of the non-electric nebulizer system. The user has connected the non-electric nebulizer system to the external nebulizer mask via a pipe for the delivery of the medicine. Further, the nebulizer works independently of any source of electric power.

In an embodiment, the non-electric nebulizer system gets activated based on the movement of the mask outer case [108] in a clockwise direction or by pressing the disbursement trigger [114] in the mask outer case [108]. For instance, the user using the non-electric nebulizer system is required to rotate the mask outer case [108] in the clockwise direction or by pressing the disbursement trigger while using the mask of the nebulizer to the face. After a sufficient number of rotations or pressing the disbursement trigger, the non-electric nebulizer system starts to work and the user can inhale the required fluid medicines. Similarly, to deactivate the non-electric nebulizer system, the user is required to rotate the mask outer case [108] in the counter or anticlockwise direction or by releasing the embedded trigger. After a sufficient number of rotations in the anticlockwise direction, the non-electric nebulizer system stops the associated functions of spraying the medicines through the mask.

As shown in Fig. 4, the pipe connects the non-electric nebulizer to the external nebulizer mask for allowing the user to inhale fluids from the nebulizer mask in any body posture. The pipe enables the delivery of the fluids from the nebulizer tank to the external mask to allow the user to inhale the fluid in the tank through the mask in any body posture.

FIG.5 illustrates a method flow diagram [200] for inhaling fluid using a non-electric nebulizer, in accordance with the exemplary embodiment of the present disclosure.

As shown in Figure 5, the method [200] begins at step [202] following a need of using nebulizer which does not require dependency on the electric power. For instance, the user wants to use a nebulizer which can be used at any place and is independent of electric power supply. The method [200] includes a first step [204] of receiving a first input from a user for activation of the non-electric nebulizer. In a non-limiting embodiment, the first input of the user for the activation of the non-electric nebulizer is one of rotating the at least one mask outer case [108] of the non-electric nebulizer in a clockwise direction for a predefined number of rotations and pressing a disbursement trigger [114] of the non-electric nebulizer.

In an embodiment, the non-electric nebulizer gets activated based on the input of the user associated with rotating the at least one mask outer case [108] of the non-electric nebulizer in a clockwise direction for a predefined number of rotations.

In another embodiment, the non-electric nebulizer gets activated based on the input of the user associated with pressing a disbursement trigger [114] of the non-electric nebulizer.

The method [200] includes the next step [206] of activating the non-electric nebulizer by initiating the movement of the fluid from at least one fluid tank [112] to at least one mask outer case [108] using Bernoulli's Theory Enabler [110]. The mask outer case [108] of the system is connected with the disbursement trigger [114] that enables the disbursement of the high pressure of the gas or air or mixture of gases. The lower part of the disbursement trigger [114] mixes the high-pressure gases or mixture of gases from the bottle [102]. Further, the disbursement trigger [114] works by mixing the high-pressure air/gas or fluid of the bottle [102] with the pressure of the stored fluid in the fluid tank [112] to disburse the continuous or non-continuous flow of the gases or mixtures in a controlled manner. Finally, the flow of the gases or mixture moves in an upward direction from the fluid tank [112] to the mask outer case [108] for enabling the user to inhale the fluid through the mask connected with the tube pipe [116].

The method [200] includes the further step [208] of inhaling the fluid by the user using at least one mask outer case [108] of the non-electric nebulizer. In a non-limiting embodiment, the at least one pipe tube point [116] enables the user to connect the non-electric nebulizer system with an external mask to use the non-electric nebulizer system in one or more favourable positions of the user.

After taking the required dose of fluid or medicinal drug, the user wants to deactivate the non-electric nebulizer. The method [200] includes the step [210] of receiving a second input from the user for the deactivation of the non-electric nebulizer. In a non-limiting embodiment, the second input of the user for the deactivation of the non-electric nebulizer is one of rotating the at least one mask outer case [108] of the non-electric nebulizer in an anti-clockwise direction for a predefined number of rotations and releasing a disbursement trigger [114] of the non-electric nebulizer.

In an embodiment, the method [200] includes the step [212] deactivating the non-electric nebulizer by stopping the movement of the fluid from at least one fluid tank [112] based on the second input. In a non-limiting embodiment, the second input of the user for the deactivation of the non-electric nebulizer corresponds to rotating the at least one mask outer case [108] of the non-electric nebulizer in an anti-clockwise direction for a predefined number of rotations. In another embodiment, the second input of the user for the deactivation of the non-electric nebulizer corresponds to releasing of the disbursement trigger [114] of the non-electric nebulizer.

In a non-limiting embodiment, the method [200] further comprises connecting at least one external mask with the non-electric nebulizer using at least one tube point [116] to use the non-electric nebulizer system in one or more favorable positions of the user. The method [200] ends at step [214].

In an exemplary embodiment, a user X rotates the mask outer case of the non-electric nebulizer in clockwise direction. The nebulizer is activated, and the fluid is moved from fluid tank to mask outer case using Bernoulli's Theory Enabler. User X inhales the fluid using an external mask attached to the tube point while lying on the bed. After inhaling the fluid for a while, user X rotates the mask outer case of the non-electric nebulizer in an anti-clockwise direction and the nebulizer is deactivated.

In another exemplary embodiment, a user Y presses the disbursement trigger of the non-electric nebulizer. The nebulizer is activated, and the fluid is moved from fluid tank to mask outer case using Bernoulli's Theory Enabler. User Y inhales the fluid using an external mask attached to the tube point while sitting on the chair. After inhaling the fluid for a while, user Y releases the disbursement trigger of the non-electric nebulizer, and the nebulizer is deactivated.

As evident from the above disclosure, the present solution provides significant technical advancement over the existing solutions by enabling the functioning of the nebulizer system without electric power. Further, the inbuilt integrated spray mechanism for the flow of gas or mixture of gases or air without electric power enables the user to use the nebulizer system anywhere and at any time. The user will be able to inhale the fluid through the nebulizer system without plugging the nebulizer system into an electrical socket thereby ensuring the most efficient use of the nebulizer, not only indoors, but also outdoors and on the go. Furthermore, the user will be able to use the nebulizer system for the inhalation of fluids from the nebulizer mask in any body posture of his choice with the help of the tube pipe that connects the non-electric nebulizer system to the external nebulizer mask.

While considerable emphasis has been placed herein on the disclosed embodiments, it will be appreciated that many embodiments can be made and that many changes can be made to the embodiments without departing from the principles of the present disclosure. These and other changes in the embodiments of the present disclosure will be apparent to those skilled in the art, whereby it is to be understood that the foregoing descriptive matter to be implemented is illustrative and non-limiting.

## Claims

1. A non-electric nebulizer system (100) comprising:
- a container (102) containing a mixture of gases;
- a valve (104) air tightly positioned at top of the container (102) to hold the mixture of gases under a compressed pressure;
- a mask holder (106) positioned on top of the valve (104) to hold a mask outer case (108);
- the mask outer case (108) positioned at top of the mask holder (106) to facilitate movement of the mask outer case (108) in a clockwise and an anti-clockwise direction;
- a fluid tank (112) positioned on the top of the mask holder (106) to store and dispense a medicinal fluid;
- a Bernoulli's Enabler (110) positioned inside the fluid tank (112) to release the mixture of gases for dispensing the medicinal fluid;
- a disbursement trigger (114) fixed on top of the mask outer case (108); and
- a pipe tube point (116) to connect the non-electric nebulizer system (100) with an external mask;
wherein the non-electric nebulizer system (100) is activated based on one of a movement of the mask outer case (108) in a clockwise direction for a predefined number of rotations and pressing the disbursement trigger (114).

2. The system (100) as claimed in claim 1, wherein the non-electric nebulizer system (100) is deactivated based on one of movement of the mask outer case (108) in an anti-clockwise direction for the predefined number of rotations and by releasing the disbursement trigger (114).

## Patentansprüche

1. Nicht-elektrisches Verneblersystem (100), aufweisend:
- einen Behälter (102), der ein Gasgemisch enthält;
- ein Ventil (104), das an der Oberseite des Behälters (102) luftdicht positioniert ist, um das Gasgemisch unter einem komprimierten Druck zu halten;
- einen Maskenhalter (106), der auf der Oberseite des Ventils (104) positioniert ist, um ein Maskenaußengehäuse (108) zu halten;
- das Maskenaußengehäuse (108), das an der Oberseite des Maskenhalters (106) positioniert ist, um eine Bewegung des Maskenaußengehäuses (108) im Uhrzeigersinn und im Gegenuhrzeigersinn zu erleichtern;
- einen Fluidtank (112), der auf der Oberseite des Maskenhalters (106) positioniert ist, um ein medizinisches Fluid zu lagern und abzugeben;
- einen Bernoulli's Enabler (110), der innerhalb des Fluidtanks (112) positioniert ist, um das Gasgemisch zum Abgeben des medizinischen Fluids freizugeben;
- einen Ausgabeauslöser (114), der auf der Oberseite des Maskenaußengehäuses (108) befestigt ist; und
- einen Rohrleitungspunkt (116), um das nicht-elektrische Verneblersystem (100) mit einer externen Maske zu verbinden;
wobei das nicht-elektrische Verneblersystem (100) basierend auf einem von einer Bewegung des Maskenaußengehäuses (108) im Uhrzeigersinn für eine vordefinierte Anzahl von Drehungen und einem Drücken des Ausgabeauslösers (114) aktiviert wird.

2. System (100) nach Anspruch 1, wobei das nicht-elektrische Verneblersystem (100) basierend auf einem von einer Bewegung des Maskenaußengehäuses (108) im Gegenuhrzeigersinn für die vordefinierte Anzahl von Drehungen und durch Freigeben des Ausgabeauslösers (114) deaktiviert wird.

## Revendications

1. Système de nébuliseur non électrique (100) comprenant :
- un récipient (102) contenant un mélange de gaz ;
- une valve (104) positionnée de manière étanche à l'air au niveau d'un sommet du récipient (102) pour maintenir le mélange de gaz sous une pression de compression ;
- un support de masque (106) positionné sur un sommet de la valve (104) pour maintenir un boîtier extérieur de masque (108) ;
- le boîtier extérieur de masque (108) positionné au niveau d'un sommet du support de masque (106) pour faciliter un mouvement du boîtier extérieur de masque (108) dans un sens horaire et un sens anti-horaire ;
- un réservoir de fluide (112) positionné sur le sommet du support de masque (106) pour stocker et administrer un fluide médicinal ;
- un activateur de Bernoulli (110) positionné à l'intérieur du réservoir de fluide (112) pour libérer le mélange de gaz afin d'administrer le fluide médicinal ;
- un déclencheur de dispersion (114) fixé sur un sommet du boîtier extérieur de masque (108) ; et
- un point de raccordement tubulaire (116) pour raccorder le système de nébuliseur non électrique (100) à un masque externe ;
dans lequel le système de nébuliseur non électrique (100) est activé sur la base de l'un parmi un mouvement du boîtier extérieur de masque (108) dans le sens horaire pour un nombre prédéfini de rotations et une pression sur le déclencheur de dispersion (114).

2. Système (100) selon la revendication 1, dans lequel le système de nébuliseur non électrique (100) est désactivé sur la base de l'un parmi un mouvement du boîtier extérieur de masque (108) dans le sens anti-horaire pour le nombre prédéfini de rotations et la libération du déclencheur de dispersion (114).
